# NOUVEAU FASCICULE DE BREVET EUROPEEN

(11) **EP 0 986 532 B2**
(45) Date de publication et mention de la décision concernant l'opposition: **25.10.2006**
(45) Mention de la délivrance du brevet: 30.07.2003
(21) Numéro de dépôt: 98923941.3
(22) Date de dépôt: 05.06.1998
(51) Int. Cl.: C07C 51/42, C07C 51/44, C07C 51/47, C07C 59/08

(54) **PROCEDE DE PURIFICATION D'ACIDE LACTIQUE**
VERFAHREN ZUR REINIGUNG VON MILCHSÄURE
METHOD FOR PURIFYING LACTIC ACID

(30) Priorité: 06.06.1997 BE 9700489
(43) Date de publication de la demande: 22.03.2000
(73) Titulaire: Brussels Biotech, 1180 Bruxelles (BE)
(72) Inventeur: VAN GANSBERGHE, Frédéric, B-1170 Bruxelles (BE); BOGAERT, Jean-Christophe, B-9308 Gijzegem (BE); MALHAIZE, Etienne, B-1341 Céroux-Mousty (BE); VAN GANSBERGHE, Martin, B-13410 La Hulpe (BE); WOLFF, Florence, B-1190 Bruxelles (BE)
(74) Mandataire: Vandeberg, Marie-Paule L.G.
(86) Numéro de dépôt international: PCT/BE1998/000080
(87) Numéro de publication internationale: WO 1998/055442

(56) Documents cités:
- DE-A- 203 533
- FR-A- 1 290 212
- FR-A- 2 067 972
- JP-A- 191 788
- JP-A- 5 112 990
- US-A- 1 594 843
- R. Leenaerts, Revue Energie Primaire, 8(2), p. 49-80 (1972)

## Description

L'acide lactique ou acide hydroxy-2-propanoique est un acide carboxylique α-hydroxylé qui peut être produit par fermentation de divers substrats carbonés purs ( glucose, saccharose, lactose, ...) ou impurs ( produits d'hydrolyse de l'amidon, mélasses, lactosérum,... ) à l'aide de microorganismes tels que les bactéries des genres Lactobacillus, Pediococcus, Lactococcus et Streptococcus, ou certains champignons tels que les Rhizopus Oryzae. D'autres voies d'obtention de l'acide lactique sont connues de l'homme de l'art, via des transformations chimiques de réactifs issus de la pétrochimie telles que l'hydrolyse du lactonitrile lui même obtenu au départ d'acétaldéhyde, la chloration et l'hydrolyse d'acide propionique ou encore via la nitration du propène.

L'acide lactique existe sous deux formes diastéréo-isomèriques : acide L(+) et D(-) lactique et rencontre chaque jour de nouvelles applications, depuis l'utilisation classique en tant que conservant alimentaire jusqu'à de nouveaux développements tels que la synthèse de solvants, pesticides, herbicides, polymères biodégradables, ...Cependant, en raison du renforcement croissant des critères de qualité requis et de la nécessité d'atteindre des coûts de production compatibles avec le marché, il est crucial de développer des techniques de purification efficaces et peu coûteuses.

L'acide lactique peut être purifié soit par précipitation sous forme de lactates métalliques suivie d'une réaction de neutralisation à l'acide sulfurique ( Maesato K., Komori A., Taki Chem. Co, JP 6,272,646 (25/09/85)), soit par estérification avec un alcool, distillation et hydrolyse de l'esterformé (BorodaT.A., Polovko V.N., Chistyakova E.A., Pishch. Prom. 1966, 4, 35-8), soit par électrodialyse ( Jacquement J.C., Rhone-Poulenc, DE 1,957,395 (14/11/68)) Ces procédés souffrent dans le premier cas, d'une piètre qualité du produit et de fortes pertes en acide lactique, tandis que dans les deux autres cas le coût est prohibitif. Un procédé de purification plus récent consiste à extraire l'acide lactique par extraction liquide-liquide au moyen d'au moins un solvant organique non miscible à l'eau, en présence ou non d'au moins une base de Lewis telle qu'une amine tertiaire. Selon ce procédé, l'acide lactique doit être récupéré dans une seconde étape par une extraction liquide-liquide en retour. Cette étape permet de retransférer l'acide lactique dans l'eau.(Baniel A.M., Blumberg R., Hadju K.,IMI, DE 2,329,480 (19/06/72); Baniel A.M., Miles Lab., EP 49,429 (06/10/80)). Enfin, l'acide lactique sous forme acide et/ou sous forme de lactate d'ammonium ou encore de lactate métallique peut être purifié par passage sur des colonnes échangeuses d'ions cationiques et/ou anioniques (Napierala W., Siminski M., Przem. Ferment. Rolny. 1972,16(12), 4-10 ; Shkurino O.V., Dauksha V.E., Khim-Farm.Zh. 1986, 20(10), 1375-77 ; Maesato K., Komori A., Taki Chem.Co., JP 6,272,646 (25/09/85); Obara H.,Shimadzu corp., JP 63,188,632 (30/01/87) ; Obara H., Shimadzu Corp, JP 0,191,788 (30/09/87) ; Zeleneva N.A., Ivanova E.V., Karpushina I.A., Gaevskaya M.V., Teoriya I Prakitika Sorbtsionnykl Protsessov, 1982, 67-69).

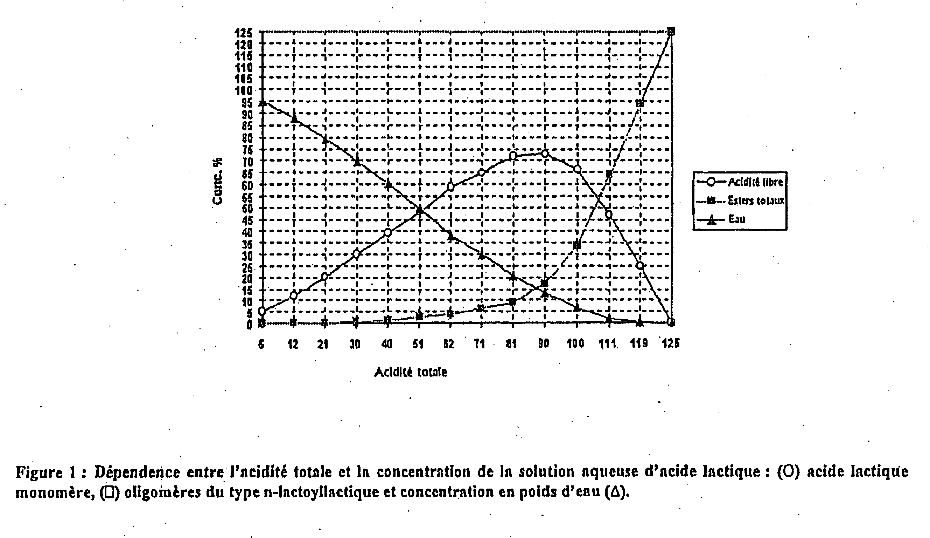

Il est à noter que toutes ces opérations de purification s'effectuent généralement au départ de solutions diluées d'acide lactique dans l'eau. Ceci se justifie par la structure même de l'acide lactique porteur à la fois d'une fonction hydroxyle et d'un groupe acide carboxylique. En effet, la bifonctionnalité de l'acide lactique est à l'origine de réactions de condensation générant des unités lactoyllactique, dilactoyllactique, trilactoyllactique, ... (n-lactoyllactique) aussi appelées oligomères d'acide lactique. Ces réactions de condensation ou d'oligomérisation tendent vers un équilibre, mais sont d'autant plus probables que la concentration de la solution aqueuse de départ est élevée (Holten C.H., « Lactic acid : Properties and chemistry of lactic acid and derivatives », Verlag Chemie, 1971). La figure 1 montre l'équilibre qui existe entre la forme monomère de l'acide lactique et les oligomères dans toute la gamme de concentration envisageable.

Les réactions de condensation ou d'oligomérisation de l'acide lactique correspondant en fait à des réactions d'estérification, elles sont catalysées par des acides et des bases de Brônsted et de Lewis. Dés lors dans le but d'éviter ou de minimiser l'intervention de ces réactions, il est impératif d'éliminer toute trace d'impuretés susceptibles de catalyser l'oligomérisation au moyen d'une étape de purification préalable. Par ailleurs, il est aussi reconnu que la température accélère la formation d'oligomères (Holten C.H., « Lactic acid : Properties and chemistry of lactic acid and derivatives »,Verlag Chemie, 1971 ).Ceci explique pourquoi l'acide lactique en solution aqueuse a longtemps été considéré comme une substance peu volatile et indistillable à 100°C. En fait, l'acide lactique se condense pour former des oligomères dont le point d'ébullition est supérieur à 100°C sous pression atmosphérique. Des travaux plus récents sur la distillation de l'acide lactique par entraînement à la vapeur à 160-200°C montrent qu'il est possible de le distiller avec des rendements de l'ordre de 75 à 85%. Néanmoins, ces conditions drastiques sont préjudiciables à la qualité du produit; dégradation et racémisation sont impossible à éviter. Une variante à la distillation par entraînement de vapeur d'eau a été proposée par Noerdlinger (U.S. 924,494, DE 221,786, et DE 224,664). Cette technique consiste au passage à grande vitesse d'air ou de gaz inerte chaud en surface d'une solution d'acide lactique préalablement débarrassée de l'eau qu'elle contient. La consommation énergétique et la faiblesse des rendements obtenus rendent cependant l'opération peu viable industriellement. Pour être complet, signalons que d'autres profils d'appareil et dispositions ont été rapportés, qui permettent, avec plus ou moins de succès, de concentrer et de distiller l'acide lactique en solution diluée dans l'eau sous pression réduite dans un évaporateur présentant une très grande surface de vaporisation par rapport au volume de liquide engagé (Sepitka A., Prumisl Potravin 13, 385 et 605 (1962) et 14, 45 et 82 (1963); ShishkiniA.V., Domanskii I.V., U.S.S.R. 709,613 (10/05/77)).

Dans le document US-1 594 843, on décrit un procédé pour la récupération et la purification d'une solution aqueuse d'acide lactique. Selon ce procédé connu, on soumet la solution aqueuse à une vaporisation instantanée de la totalité de l'acide lactique, dans le but d'éviter la formation d'anhydride lactique et de lactide.

Ce procédé connu ne conduit toutefois pas à de l'acide lactique de pureté suffisante.

L'invention vise à remédier à ce désavantage du procédé connu, en fournissant un procédé nouveau, permettant d'obtenir de l'acide lactique de grande pureté, avec un rendement massique particulièrement élevé.

A cet effet, selon l'invention, avant la distillation, on soumet la solution successivement à un prétraitement permettant d'éliminer les substances ioniques capables de catalyser la polycondensation de l'acide lactique et à deux étapes de concentration qui sont réglées pour éliminer la totalité de l'eau libre de ladite solution, la distillation étant effectuée de manière sélective et essentiellement quantitative. Par quantitative, on entend que l'entièreté de la fraction distillable est effectivement distillée. Par sélective, on entend que seul le monomère (et, dans une moindre proportion, le dimère) de l'acide lactique distille(nt) sans entraîner d'impuretés ou de produits de dégradation.

La présente invention consiste en un procédé de purification de l'acide lactique en solution aqueuse tel qu'obtenu d'un milieu de fermentation ou de toute autre source préalablement débarrassée des composés solides et/ou de la biomasse. En ce qui concerne l'étape de séparation des composés solides, il est fait référence à toute méthode connue de l'homme de l'art telle que la centrifugation, la floculation, la microfiltration, ....Par contre, le procédé de purification décrit dans cette invention est original en ce sens qu'il assure l'obtention d'une très haute qualité d'acide lactique avec un rendement massique particulièrement élevé et une consommation énergétique minimale. On entend par très haute qualité, des concentrations résiduelles en impuretés minérales et organiques telles que l'acide lactique purifié peut être utilisé pour des applications pharmaceutiques en conformité avec toutes les Pharmacopae actuelles. L'acide lactique purifié par le procédé décrit dans la présente invention est de plus thermostable, c'est à dire q u'il reste incolore après un traitement thermique de 2 heures à 180°C, et conserve l'activité optique de l'acide lactique engagé (procédé stéréospécifique ).On entend par rendement massique, le rapport exprimé en pourcent, de la masse d'acide lactique purifié sur la masse d'acide lactique engagé ; ces masses correspondants à des concentrations en acide lactique de 100%. Il est bien entendu que les applications requérant une pureté moindre peuvent aussi être satisfaites par la technique proposée dans la présente invention. Les aspects quantitatif et sélectif de ce procédé de purification sont assurés par la mise en oeuvre conjointe (1) d'un prétraitement visant l'élimination des substances susceptibles de catalyser la réaction de condensation de l'acide lactique, (2) des conditions de température, de temps de séjour et de viscosité permettant de réduire l'intervention de ces mêmes réactions de condensation, et (3) des conditions de température, de temps de séjour, de viscosité, de pression et de profil d'appeillage rendant possible la concentration jusqu'à atteindre une concentration en poids de 100%, et la distillation de l'acide tactique.

### Description de l'invention

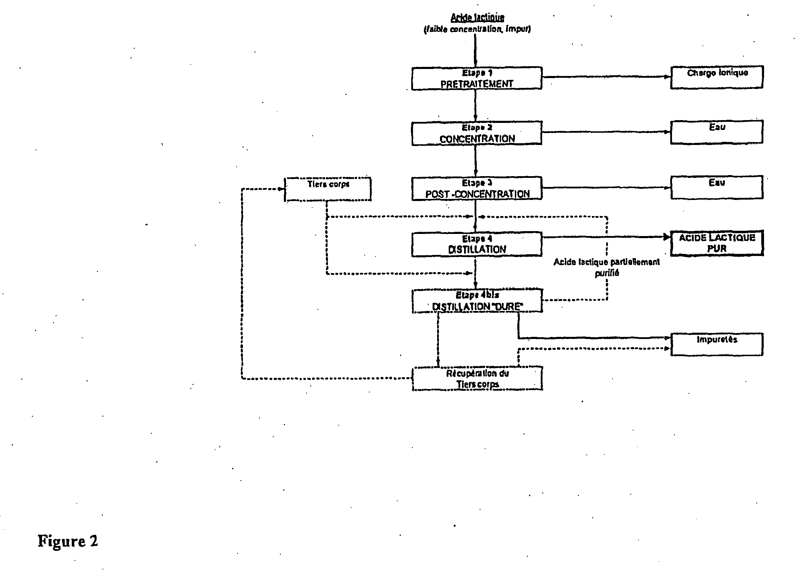

La présente invention décrit un procédé de purification de l'acide lactique provenant d'une solution aqueuse de cet acide telle qu'obtenue d'un milieu de fermentation ou de toute autre source préalablement débarrassée des substances solides et/ou de la biomasse éventuellement présentes. La figure 2 illustre le procédé de purification de l'acide lactique tel qu'il y estfait référence dans la présente invention. Ce procédé comprend essentiellement les étapes suivantes :

### 1. Prétraitement de la solution diluée d'acide lactique (1)

Le prétraitement considéré dans le cadre de l'invention consiste en l'élimination des substances ioniques capables de catalyser la condensation ou oligomèrisation de l'acide lactique. Ce prétraitement s'effectue à faible concentration en acide lactique, soit à une concentration inférieure à 80%, préférablement inférieure à 50% et plus préférablement encore inférieure à 30%. Une approche privilégiée de la présente invention consiste à éliminer les substances ioniques au moyen de résines échangeuses d'ions. Ainsi, la mise en contact de la solution d'acide lactique avec une résine échangeuse d'anions, préalablement conditionnée sous forme basique (OH-), permet d'échanger les impuretés anioniques contenues dans la solution traitée par des groupes hydroxydes. La présente invention ne s'arrête pas à l'utilisation de résines échangeuses d'anions solides, mais comprend toute autre technique connue de l'homme de l'art permettant l'élimination des charges anioniques au profit d'ions hydroxydes, telle que l'utilisation d'amines grasses quaternisées et présentes sous forme d'hydroxyde d'ammonium en solution dans au moins un solvant organique non miscible avec l'eau. Dans ce cas, l'échange anion/hydroxyde a lieu à l'interface des phases non miscibles et est suivi d'une séparation des phases. Une approche préférée de l'invention consiste à faire précéder l'étape d'échange d'anions par un traitement qui se caractérise en ce que la solution d'acide lactique est débarrassée des charges cationiques, mono-, di-, tri-, et/ou multivalentes, éventuellement présentes. Les impuretés cationiques sont éliminées par mise en contact avec une résine échangeuse de cations prélablement conditionnée en milieu acide (H⁺). Cette approche est préférée dans la mesure ou elle permet d'éviter la formation et la précipitation d'hydroxydes métalliques peu solubles dans l'eau lors du traitement anionique. Ici encore, l'invention ne se limite pas aux résines échangeuses de cations mais s'étend à toute autre technique connue de l'homme de l'art capable d'échanger les cations de la solution d'acide lactique au profit de protons. Il est, par exemple, fait référence à l'utilisation d'acide gras de type carboxylique ou sulfonique en solution dans au moins un solvant organique non miscible à l'eau. L'échange cation/proton a lieu à l'interface entre solvants non miscibles et est suivi d'une séparation des phases.

### 2. Concentration de la solution d'acide lactique (2)

Cette étape de l'invention consiste en la concentration rapide et à basse température de la solution d'acide lactique, préalablement traitée selon la méthode reprise comme première étape de l'invention (1), jusqu'à atteindre une concentration comprise entre 50 et 90%, préférablement entre 70 et 90%. Une approche préférée de la présente invention envisage la conduite de cette évaporation sous pression réduite, maintenue entre 50 et 500 mbars absolus et préférablement entre 50 et 250 mbars, afin d'assurer une température d'ébullition de la solution aussi basse que possible. Cette étape de l'invention est réalisée par toute technique connue de l'homme de l'art, telle que l'évaporation en film ruisselant.

### 3. Post-concentration de la solution d'acide lactique (3)

Cette étape permet la post-concentration de la solution effluant de l'appareillage réservé à l'étape (2) jusqu'à une concentration de 100% en acide lactique. L'opération peut avantageusement être réalisée, avec un temps de séjour minimal et à une température aussi basse que possible, dans un appareil à film mince mécaniquement agité (thin-film evaporator) ou à l'aide d'un évaporateur à court trajet (short-path evaporator). La pression est de l'ordre de 10 à 500 mbars, préférablement comprise entre 50 et 300 mbars et plus préférablement encore comprise entre 50 et 150 mbars. La température de la paroi chauffante du corps de l'évaporateur est ajustée de manière à supporter la vaporisation de l'eau libre contenue dans la solution à concentrer sans pour autant surchauffer cette dernière ; soit une température comprise entre 50 et 150°C, préférablement entre 80 et 120°C. De manière surprenante, il a été observé que si l'acide lactique se présente quantitativementsous forme de monomère d'acide lactique ( et en l'absence d'eau libre concentration = 100% ), il est possible de le distiller sous pression réduite dans un réacteur maximisant la surface de vaporisation par rapport au volume de liquide. Outre de revendiquer l'utilisation d'un tel profil de réacteur pour la distillation de l'acide lactique, la présente invention garanti l'obtention quantitative de cet acide concentré sous forme de monomère distillable avant sa purification proprement dite par distillation.

### 4. Purification de l'acide lactique par distillation (4)

Cette étape se caractérise en ce que la solution déminéralisée et concentrée d'acide lactique, telle que produite dans les étapes (1) à (3), est soumise à des conditions telles que le monomère (et dans une moindre proportion le dimère) de cet acide distille(nt) de manière quantitative et sélective. Par quantitative, on entend que l'entièreté de la fraction distillable est effectivement distillée. Par sélective, on entend que seul le monomère (et dans une moindre proportion le dimère ) de l'acide lactique distille(nt), sans entraîner d'impuretés ou de produits de dégradation. Cette étape est avantageusement conduite dans un réacteur maximisant la surface de vaporisation par rapport au volume de liquide, c'est à dire par un réacteur exploitant les propriétés de la couche mince. Une approche préférée de la présente invention consiste à utiliser pour la distillation de l'acide lactique 100% un évaporateur à film mince mécaniquement agité à l'extérieur duquel l'acide lactique purifié est condensé (thin-film evaporator) ou un évaporateur à court trajet avec un condenseur interne (short-path evaporator ). Il est bien connu de l'homme de l'art qu'un tel système permet de maximiser la surface d'échange thermique et la surface de vaporisation. La température de la paroi est maintenue entre 50 et 180°C, préférablement entre 80 et 160°C, plus preférablement encore entre 110 et 160°C. La pression est comprise entre 10⁻³ et 10⁺² mbars absolus, préférablement entre 10⁻¹ et 2.10⁺¹ mbars absolus, plus préférablement encore entre 1 et 10 mbars. Une approche préférée de la présente invention considère une disposition verticale de l'évaporateur permettant une progression du film sous l'impulsion combinée de l'agitation mécanique et de la gravité. Selon une variante améliorée, mais non essentielle de la présente invention, le résidu de purification peut être dirigé vers un second distillateur dans lequel les conditions de température et de pression sont plus drastiques ( figure 2, étape 4bis ). L'acide lactique issu de cette post-distillation et partiellement purifié peut être recyclé soit vers l'alimentation du distillateur principal ( étape 4 ), soit en amont du procédé. Une version préférée de la présente invention considère l'ajout d'un tiers corps destiné à faciliter l'écoulement en couche mince et l'évaporation de l'acide lactique lors de l'étape de distillation et/ou de post-distillation. Ce tiers corps comprend toute substance non-toxique, chimiquement inerte vis-à-vis de l'acide lactique, de faible volatilité, thermostable et de faible viscosité dans les conditions de distillation et de post-distillation, et préférablement immiscible à l'acide lactique afin d'en faciliter la séparation par décantation et le recyclage. A titre d'exemple, mentionnons que l'utilisation de paraffines telles que Fina Vestan A80B, A180B et préférablement A360B, ont permis de favoriser le drainage des impuretés et l'évaporation de l'acide lactique, tout en répondant aux exigences précitées. D'autres détails et particularités de l'invention, donnés ci-après à titre d'exemples non-limitatifs, ressortent de la description comme quelques formes possibles de sa réalisation.

### EXEMPLES

### Exemple 1

### Déminéralisation d'une solution d'acide lactique.

Une solution d'acide lactique obtenue par fermentation est déminéralisée par percolation sur résines solides d'échange ionique. La solution alimentée présente l'analyse suivante: acide lactique 185.1 g.l⁻¹, pH 2.25, sulfates 1250 ppm, calcium 929 ppm, fer 15.8 ppm, potassium 133 ppm et sodium 98 ppm. Cette solution est alimentée à raison de 3 BV/h sur le dessus d'une colonne contenant 1 BV de résine macroporeuse cationique forte de structure polystyrène réticulé BAYER Lewatit S 2528 préalablement conditionnée sous forme H⁺ par passage de 120 g d'acide chlorhydrique pur par litre de résine sous forme d'une solution à 6%. L'effluent récolté en sortie de cette colonne est ensuite dirigé vers une colonne contenant le même volume de résine anionique de basicité moyenne formée de groupements amines ternaires et quaternaires greffés sur une structure polystyrène commercialisée par BAYER sous la référence Lewatit S 4328. Cette résine est préalablement conditionnée sous forme basique par percolation de 120 g de soude caustique pure sous la forme d'une solution à 4% de concentration. La solution d'acide lactique traitée dans ces conditions présente l'analyse moyenne suivante après traitement d'un volume de solution correspondant à 15 fois le volume de résine cationique : acide lactique 167 g.l⁻¹, pH 1.75, sulfates 0.7 ppm, calcium 0.8 ppm, fer 0.3 ppm, potassium 1.1 ppm et sodium 0.9 ppm. Le perçage de la résine cationique qui se manifeste par une augmentation de la concentration en cations monovalents dans l'effluent de la première colonne du système est survenu après passage de 15 BV de solution d'acide lactique. Le perçage de la résine anionique, mis en évidence par détection d'ions sulfates dans l'effluant de la seconde colonne du système, est survenu après traitement de 18 BV de solution alimentée.

### Exemple 2

### Concentration d'une solution d'acide lactique jusqu'à 80%.

Une solution traitée suivant l'exemple 1 est alimentée en continu dans un évaporateur à film ruisselant en inox présentant une surface d'évaporation de 0.31 m². La solution concentrée d'acide lactique est extraite au même débit que le débit d'alimentation du système (10.45 l.h⁻¹) afin d'y maintenir un niveau constant. Le chauffage de la paroi est assuré par une circulation d'huile caloporteuse dans une double enveloppe. Les conditions de pression, de température, ainsi que les concentrations obtenues sont présentées dans le tableau 1 repris ci-dessous.

**Tableau 1 :**

| Concentration d'une solution aqueuse d'acide lactique 18.5% en pds dans un évaporateur de type film ruisselant de 0.31 m² de surface d'évaporation. | | | | |
|---|---|---|---|---|
| Pression (mbars) | Température (°C) | Concentration visée (% pds) | Concentration⁽¹⁾ obtenue (% pds) | Δconcentration (% pds) |
| 98 | 46.9 | 70 | 68.3 | -1.7 |
| 102 | 47.2 | 70 | 71.7 | 1.7 |
| 204 | 68.3 | 70 | 70.2 | 0.2 |
| 100 | 21.8 | 75 | 74.4 | -0.6 |
| 103 | 56.4 | 80 | 79.6 | -0.4 |
| 101 | 69 | 85 | 84.6 | -0.4 |
| 197 | 82.6 | 85 | 86.5 | 1.5 |
| 96 | 68.3 | 85 | 82.1 | -2.9 |

| | | | | |
|---|---|---|---|---|
| ⁽¹⁾ la concentration ou acidité totale est déterminée par titrage acido-basique après saponification. | | | | |

### Exemple 3

### Post-concentration d'acide lactique à diverses pressions.

Une solution d'acide lactique 81.75% en pds (taux de polymérisation = 13.19%) est alimentée en continu dans un évaporateur en verre borosilicate à film mince mécaniquement agité avec condenseur interne (short-path) de marque UIC présentant des surfaces de chauffage et de condensation de 0.06 m², précédé d'un dégazeur-préchauffeur dont la température est ajustée par circulation d'huile caloporteuse. L'ensemble est maintenu sous une pression de 50 à 250 mbars absolus. Les résultats présentés au tableau 2 ont été obtenus avec une température de paroi de 100°C, une température de dégazage de 80°C, une température de condenseur de 15°C, une vitesse de rotation du rotor de 400 rpm (rotations par minute) et un débit d'alimentation de 1000 g.h⁻¹.

**Tableau 2 :**

| Post-concentration d'une solution d'acide lactique 81.75% en pds dans un évaporateur type short-path UIC de 0.06 m2. | | | | |
|---|---|---|---|---|
| Pression (mbars) | Acidité totale⁽¹⁾concentrats (% pds) | Acidité totale⁽¹⁾condensats (% pds) | Taux de⁽²⁾polymérisation concentrats (%) | Rendement (%) |
| 50 | 100.8 | 46.1 | 15.5 | 70.7 |
| 100 | 101.8 | 12.1 | 14.4 | 96.2 |
| 150 | 101.5 | 8.4 | 14.2 | 97.9 |
| 200 | 99.7 | 6.9 | 12.9 | 95.9 |
| 250 | 100.2 | 6.5 | 13.6 | 97.6 |

| | | | | |
|---|---|---|---|---|
| ⁽¹⁾ l'acidité totale est déterminée par titrage acido-basique après saponification. ⁽²⁾ Le taux de polymérisation se définit comme le rapport de l'acidité estérifiée (pourcentage en poids de groupe acide carboxylique sous forme ester) sur l'acidité totale. | | | | |

### Exemple 4

### Influence du séjour à chaud sur le taux de polymérisation (en système statique).

Une solution d'acide lactique déminéralisée telle qu'obtenue aux exemples 1, 2 et 3, et présentant une concentration de 98.1 % en poids (taux de polymérisation =13.1 %), est maintenue à 100°C et pression atmosphérique durant un temps variante. Le tableau 3 montre la dépendance du taux de polymérisation en fonction du temps de séjour.

**Tableau 3 :**

| Influence du temps de séjour à chaud sur le taux de polymérisation d'une solution d'acide lactique d'une acidité totale de 98.1% en pds. | | |
|---|---|---|
| Temps de séjour (min) | Acidité libre⁽¹⁾ (% pds) | Taux de polymérisation⁽²⁾(%) |
| 0 | 85.9 | 12.5 |
| 60 | 83.9 | 14.5 |
| 120 | 81.7 | 16.7 |
| 240 | 74.9 | 23.7 |
| 1050 | 65.0 | 33.8 |
| 1395 | 64.0 | 34.8 |

| | | |
|---|---|---|
| ⁽¹⁾ l'acidité libre est déterminée par titrage acido-basique. ⁽²⁾ Le taux de polymérisation se définit comme le rapport de l'acidité estérifiée sur l'acidité totale soit 98.1 %pds. | | |

### Exemple 5

### Influence du temps de séjour sur le taux de polymérisation (en système dynamique)

Une solution d'acide lactique de concentration égale à 102% en pds obtenue de manière similaire à celle décrite dans les trois premiers exemples de la présente invention est alimentée à débit constant dans un évaporateur en verre borosilicate à film mince mécaniquement agité avec un condenseur interne (short-path) de marque UIC présentant des surfaces de chauffage et de condensation de 0.06 m². Le système est maintenu sous une pression de 40 mbars absolus, la température du condenseur et la température de paroi sont respectivement ajustées à 18°C et à 160°C (tableau 4 ). On assume ici que, tous autres paramètres restant constants, le temps de séjour dans l'appareil au contact de la paroi chauffée augmente lorsque le débit d'alimentation diminue.

**Tableau 4 :**

| Influence de la température sur le taux de polymérisation d'une solution d'acide lactique de 102 % en pds dans un évaporateur de type short-path UIC 0.06m². | | | | | | |
|---|---|---|---|---|---|---|
| Débit (g/h) | Acidité totale (% pds) | % n-mère⁽¹⁾(%) | | | | |
| | | Mono- | Di- | Tri- | Tetra- | Penta- |
| 510 | 111.9 | 2.9 | 50.4 | 33.1 | 10.7 | 2.9 |
| 740 | 112.1 | 3.1 | 57.0 | 28.5 | 8.8 | 2.6 |
| 870 | 109.7 | 3.4 | 60.9 | 27.0 | 8.6 | 0.0 |

| | | | | | | |
|---|---|---|---|---|---|---|
| ⁽¹⁾ le pourcentage des n-mères est déterminé par chromatographie d'exclusion de volume (GPC). | | | | | | |

### Exemple 6 :

### Influence de la température sur le taux de polymérisation.

Une solution d'acide lactique de concentration égale à 102% en poids obtenue de manière similaire à celle décrite dans les trois premiers exemples de la présente invention est alimentée à débit constant dans un évaporateur en verre borosilicate à film mince mécaniquement agité avec un condenseur interne (short-path) de marque UIC présentant des surfaces de chauffage et de condensation de 0.06 m². Le système, alimenté à raison de 730 g.h⁻¹ est maintenu sous une pression de 40 mbars absolus. La température du condenseur est elle maintenue à 18°C (tableau 5).

**Tableau 5**

| Influence de la température sur le taux de polymérisation dans un évaporateur shorth-path UIC 0.06 m². | | | | | | |
|---|---|---|---|---|---|---|
| Température (°C) | Acidité totale (% pds) | % n- mère⁽¹⁾(%) | | | | |
| | | Mono- | Di- | Tri- | Tétra- | Penta- |
| 140 | 108.5 | 17.4 | 52.3 | 21.7 | 8.5 | 0.0 |
| 150 | 107.8 | 7.1 | 62.8 | 21.9 | 6.1 | 2.4 |
| 160 | 109.3 | 3.1 | 57.0 | 28.5 | 8.8 | 2.6 |

| | | | | | | |
|---|---|---|---|---|---|---|
| ⁽¹⁾ Le pourcentage de n-mère est déterminé par chromatographie d'exclusion de volume (GPC) | | | | | | |

### Exemple 7

### Distillation d'acide lactique et influence du taux de polymérisation sur le rendement de distillation et la qualité du distillat.

Les solutions d'acide lactique telles qu'obtenues de l'exemple 4 sont introduites à débit constant dans un évaporateur en verre borosilicate à film mince mécaniquement agité avec condenseur interne (short-path) de marque UIC présentant des surfaces de chauffage et de condensation de 0.06 m², précédé d'un dégazeur-préchauffeur dont la température est ajustée par circulation d'huile caloporteuse. L'ensemble est maintenu sous une pression de 5 mbars absolus. Le tableau 6 reprend les résultats obtenus avec une température de paroi de 140°C, une température de dégazage de 80°C, une température de condenseur de 15°C, une vitesse de rotation du rotor de 400 rpm (rotations par minute) et un débit d'alimentation compris entre 798 et 915 g.h⁻¹. La coloration des distillats obtenus est considérée comme représentative de leur pureté chimique.

**Tableau 6**

| Influence du taux de polymérisation sur le rendement de distillation et la qualité des distillats (évaporateur de type short-path UIC 0.06 m²). | | | | |
|---|---|---|---|---|
| Taux de polymérisation du fluide engagé (%) | Rendement massique (%) | Acidité totale distillats (% pds) | Taux de polymérisation distillats (%) | Coloration¹⁾ (Hazen) |
| 14.5 | 89.8 | 96.5 | 4.1 | 0 |
| | 81.5 | 97.9 | 6.5 | 0 |
| 16.7 | 82.1 | 96.8 | 5.7 | 0 |
| | 76.4 | 97.0 | 6.6 | 0 |
| 23.7 | 66.3 | 96.2 | 7.6 | 20 |
| | 64.6 | 95.3 | 7.1 | 20 |
| 33.8 | 64.8 | 96.5 | 9.5 | 70 |
| 34.8 | 43.8 | 94.9 | 9.4 | 275 |

| | | | | |
|---|---|---|---|---|
| ⁽¹⁾ La coloration est déterminée selon la norme APHA (American Public Health Association). | | | | |

### Exemple 8

### Distillation d'acide lactique et influence de la charge Ionique sur le rendement de distillation.

Une solution d'acide lactique déminéralisée telle qu'obtenue des exemples 1, 2 et 3, et présentant une concentration de 101.46% en poids, est volontairement additionnée d'acide sulfurique concentré (98%). Cette solution est ensuite introduite en continu dans un évaporateur en verre borosilicate à film mince mécaniquement agité avec condenseur interne (short-path) de marque UIC présentant des surfaces de chauffage et de condensation de 0.06 m², précédé d'un dégazeur-préchauffeur dont la température est ajustée par circulation d'huile caloporteuse (tableau 7). L'ensemble est maintenu sous une pression de 3.5 mbars absolus. Les conditions imposées au système sont les suivantes : température de paroi: 130°C, température de dégazage : 84°C, température de condenseur: 10°C, vitesse de rotation du rotor: 400 rpm (rotations par minute).

**Tableau 7**

| Influence de l'addition d'acide sulfurique à une solution d'acide lactique sur le rendement de distillation dans un évaporateur short-path UIC 0.06 m². | | | | |
|---|---|---|---|---|
| Teneur⁽¹⁾ en protons exogènes (mmol/kg) | Teneur⁽¹⁾ en ions sulfates exogènes (ppm) | Débit (g/h) | Rendement massique (%) | Entraînement⁽²⁾ au dégazeur (% pds) |
| 0 | 0 | 622 | 65.2 | 14.2 |
| | | 625 | 66.6 | |
| | | 623 | 68.0 | |
| 2.1 | 100 | 630 | 64.6 | 15.6 |
| | | 631 | 65.8 | |
| | | 630 | 69.3 | |
| 5.2 | 200 | 626 | 68.7 | 14.6 |
| | | 585 | 69.6 | |
| 8.3 | 400 | 623 | 68.0 | |
| | | 618 | 68.5 | |
| | | 622 | 69.6 | |
| 12.5 | 600 | 639 | 46.1 | 75.7 |
| | | 640 | 45.3 | |
| 24.0 | 1150 | 644 | 43.6 | - |
| | | 647 | 37.8 | |

| | | | | |
|---|---|---|---|---|
| ⁽¹⁾ On entend par exogène les ions (protons et sulfates) introduits volontairement. ⁽²⁾ L'entraînement au dégazeur signifie le pourcentage de la masse engagée qui est récoltée au dégazeur suite à des entraînements vésiculaires. | | | | |

### Exemple 9

### Distillation d'acide lactique et influence de la température sur le rendement de distillation et sur la qualité du distillat.

Une solution d'acide lactique de concentration égale à 98.12% en pds obtenue de manière similaire à celle décrite dans les trois premiers exemples de la présente invention est alimentée à débit constant dans un évaporateur en verre borosilicate à film mince mécaniquement agité avec condenseur interne (short-path) de marque UIC présentant des surfaces de chauffage et de condensation de 0.06 m². Le système, alimenté à raison de 870 g.h⁻¹, est maintenu sous une pression de 5 mbars absolus. La température du condenseur est elle maintenue à 15°C par circulation d'eau, et celle du dégazeur à 80°C par circulation d'huile caloporteuse (tableau 8).

**Tableau 8 :**

| Influence de la température sur le rendement de distillation et sur la qualité du distillat dans un évaporateur de type short-path UIC 0.06 m². | | |
|---|---|---|
| Température de paroi (°C) | Rendement massique (%) | Coloration⁽¹⁾ (Hazen) |
| 130 | 7902 | <5 |
| 140 | 82.7 | <5 |
| 150 | 88.9 | 70 |

| | | |
|---|---|---|
| ⁽¹⁾ La coloration est déterminée selon la norme APHA. | | |

### Exemple 10

### Distillation d'acide lactique et influence du temps de séjour sur la qualité du distillat.

La même solution d'acide lactique 98.12% en poids, ainsi que les mêmes conditions expérimentales qu'utilisées pour l'exemple 9, sont reprises pour deux débits d'alimentation différents, respectivement 870 et 1120 g.h⁻¹, avec une température de paroi de 150°C. Comme pour l'exemple 5, on considère que le temps de séjour dans l'appareil évolue de manière inversement proportionnelle au débit d'alimentation.

**Tableau 9**

| Influence du débit d'alimentation sur le rendement de distillation et la qualité du distillat dans un évaporateur de type short path UIC 0.06 m². | | |
|---|---|---|
| Débit d'alimentation (g/h) | Rendement massique (%) | Coloration⁽¹⁾(Hazen) |
| 870 | 88.9 | 70 |
| 1120 | 85.5 | 20 |

| | | |
|---|---|---|
| ⁽¹⁾ La coloration est déterminée selon la norme APHA. | | |

## Revendications

1. Procédé de récupération et de purification d'une solution aqueuse d'acide lactique telle qu'obtenue d'un milieu de fermentation ou de toute autre source, dans lequel on soumet la solution à une distillation de l'acide lactique, **caractérisé en ce qu'**avant la distillation, on soumet la solution successivement à un prétraitement permettant d'éliminer les substances ioniques capables de catalyser la polycondensation de l'acide lactique et à deux étapes de concentration qui sont réglées pour éliminer la totalité de l'eau libre de ladite solution, la distillation étant effectuée de manière sélective et essentiellement quantitative.

2. Procédé suivant la revendication 1, dans lequel l'acide lactique est concentré en deux étapes pour atteindre successivement, une concentration comprise entre 50 et 90 % en poids et une concentration de 100% en poids d'acide lactique, dans des conditions de pression comprise entre 50 et 500 mbars absolus.

3. Procédé suivant la revendication 1 ou 2, dans lequel la seconde étape de concentration est réalisée dans un évaporateur à film mince mécaniquement agité et muni d'un condenseur interne ou externe, sous une pression comprise entre 10 et 500 mbars et à une température de la surface chauffée comprise entre 50 et 150°C.

4. Procédé selon l'une quelconque des revendications 1 à 3, **caractérisé en ce que** la concentration et la distillation de l'acide lactique s'effectuent selon le principe de la couche mince.

5. Procédé selon l'une quelconque des revendications 1 à 4, **caractérisé en ce que** la distillation de l'acide lactique recourt à l'utilisation d'un évaporateur à film mince mécaniquement agité muni d'un condensateur interne ou externe.

6. Procédé selon l'une quelconque des revendications 1 à 5, **caractérisé en ce qu'**on effectue la distillation de l'acide lactique sous une pression comprise entre 10⁻³ et 10⁺² mbars absolus, au contact d'une surface chauffée à une température comprise entre 110 et 160 °C.

7. Procédé selon l'une quelconque des revendications 1 à 6, **caractérisé en ce que** la solution d'acide lactique est additionnée, avant la distillation, d'au moins 1 % et tout au plus 20 % par rapport à son poids, d'un composé inerte et immiscible.

8. Procédé selon l'une quelconque des revendications 1 à 7, **caractérisé en ce que** le résidu de distillation de l'acide lactique est soumis à une seconde distillation et recyclé.

9. Procédé selon la revendication 8, **caractérisé en ce que** le résidu de distillation est additionné, avant la distillation, d'au moins 1 % et tout au plus 20 % par rapport à son poids, d'un composé inerte et immiscible.

10. Procédé selon l'une quelconque des revendications 1 à 9, **caractérisé en ce que** le prétraitement de la solution d'acide lactique est effectué au départ d'une solution aqueuse d'acide lactique de concentration inférieure à 80 % en poids.

11. Procédé selon l'une quelconque des revendications 1 à 10, **caractérisé en ce que** le prétraitement de la solution d'acide lactique comprend une élimination des impuretés anionique et cationiques à l'aide d'échangeurs d'ions solides ou liquides.

## Claims

1. Process for recovering and purifying an aqueous solution of lactic acid as obtained from a fermentation medium or any other source, in which the solution is subjected to a distillation of the lactic acid, **characterized in that**, before the distillation, the solution is subjected successively to a pretreatment, which makes it possible to remove the ionic substances which are able to catalyse polycondensation of the lactic acid, and to two concentration steps which are regulated so as to remove all the free water from said solution, the distillation being effected selectively and essentially quantitatively.

2. Process according to Claim 1, in which the lactic acid is concentrated in two steps in order to reach, consecutively, a lactic acid concentration of between 50 and 90% by weight and a lactic acid concentration of 100% by weight, under pressure conditions of between 50 and 500 mbar absolute.

3. Process according to either Claim 1 or 2, in which the second concentration step is carried out in a mechanically shaken thin-film evaporator provided with an internal or external condenser, under a pressure of between 10 and 500 mbar and at a temperature of the heated surface of between 50 and 150°C.

4. Process according to any one of Claims 1 to 3, in which the lactic acid is concentrated and distilled in accordance with the thin-layer principle.

5. Process according to any one of Claims 1 to 4, in which the distillation of the lactic acid is effected using a mechanically shaken thin-film evaporator which is provided with an internal or external condenser.

6. Process according to any one of Claims 1 to 5, in which the lactic acid is distilled under a pressure of between 10⁻³ and 10⁺² mbar absolute and at a temperature of the heated surface which is between 110 and 160°C.

7. Process according to any one of Claims 1 to 6, in which the lactic acid solution is supplemented, before distillation, with at least 1%, and at the very most 20%, based on its weight, of an inert and immiscible compound.

8. Process according to any one of Claims 1 to 7, in which the residue from distilling the lactic acid is subjected to a second distillation and is recycled.

9. Process according to Claim 8, in which the distillation residue is supplemented, before distillation, with at least 1%, and at the very most 20%, based on its weight, of an inert and immiscible compound.

10. Process according to any one of Claims 1 to 9, in which the pretreatment of the lactic acid solution is effected starting with an aqueous solution of lactic acid whose concentration is less than 80% by weight.

11. Process according to either Claim 1 or 10, in which the pretreatment of the lactic acid solution includes the removal of the anionic and cationic impurities using solid or liquid ion exchangers.

## Patentansprüche

1. Verfahren zur Wiedergewinnung und Reinigung einer wässrigen Milchsäurelösung, die aus einem Fermentationsmedium oder jeder anderen Quelle erhalten wird, bei dem die Lösung einer Destillation der Milchsäure unterzogen wird, **dadurch gekennzeichnet, dass** vor der Destillation die Lösung nach und nach einer Vorbehandlung, die es ermöglicht, die ionischen Substanzen, die die Polykondensation der Milchsäure katalysieren können, zu beseitigen, und zwei Konzentrationsschritten unterzogen wird, die derart geregelt sind, dass sie die Gesamtheit des freien Wassers aus der Lösung beseitigen, wobei die Destillation auf selektive und im Wesentlichen quantitative Weise erfolgt.

2. Verfahren nach Anspruch 1, bei dem die Milchsäure in zwei Schritten konzentriert wird, um nacheinander eine Konzentration zwischen 50 und 90 Gew.-% und eine Konzentration von 100 Gew.-% Milchsäure unter Druckbedingungen zwischen 50 und 500 mbar absolut zu erreichen.

3. Verfahren nach Anspruch 1 oder 2, bei dem der zweite Konzentrationsschritt in einem mechanisch geschüttelten Dünnfilmverdampfer, der mit einem internen und einem externen Kondensator versehen ist, unter einem Druck zwischen 10 und 500 mbar und bei einer Temperatur der erhitzten Oberfläche zwischen 50 und 150 °C erfolgt.

4. Verfahren nach einem der Ansprüche 1 bis 3, **dadurch gekennzeichnet, dass** die Konzentration und die Destillation der Milchsäure nach dem Dünnschichtprinzip erfolgen.

5. Verfahren nach einem der Ansprüche 1 bis 4, **dadurch gekennzeichnet, dass** die Destillation der Milchsäure auf die Verwendung eines mechanisch geschüttelten Dünnfilmverdampfers, der mit einen internen oder externen Kondensator versehen ist, zurückgreift.

6. Verfahren nach einem der Ansprüche 1 bis 5, **dadurch gekennzeichnet, dass** die Destillation der Milchsäure unter einem Druck zwischen 10⁻³ und 10⁺² mbar absolut in Kontakt mit einer auf eine Temperatur zwischen 110 und 160 °C erhitzten Oberfläche erfolgt.

7. Verfahren nach einem der Ansprüche 1 bis 6, **dadurch gekennzeichnet, dass** der Milchsäurelösung vor der Destillation mindestens 1 % und höchstens 20 % bezogen auf ihr Gewicht einer inerten und nicht mischbaren Verbindung hinzugefügt werden.

8. Verfahren nach einem der Ansprüche 1 bis 7, **dadurch gekennzeichnet, dass** der Destillationsrest der Milchsäure einer zweiten Destillation unterzogen und rezykliert wird.

9. Verfahren nach Anspruch 8, **dadurch gekennzeichnet, dass** dem Destillationsrest vor der Destillation mindestens 1 % und höchstens 20 % bezogen auf sein Gewicht einer inerten und nicht mischbaren Verbindung hinzugefügt werden.

10. Verfahren nach einem der Ansprüche 1 bis 9, **dadurch gekennzeichnet, dass** die Vorbehandlung der Milchsäurelösung ausgehend von einer wässrigen Milchsäurelösung mit einer Konzentration von weniger als 80 Gew.-% erfolgt.

11. Verfahren nach einem der Ansprüche 1 bis 10, **dadurch gekennzeichnet, dass** die Vorbehandlung der Milchsäurelösung eine Beseitigung der anionischen und kationischen Verunreinigungen mit Hilfe von festen oder flüssigen Ionenaustauschern umfasst.
